(19) Europäisches Patentamt
European Patent Office
Office européen des brevets

(11) **EP 2 637 775 B1**

(12) **EUROPÄISCHE PATENTSCHRIFT**

(45) Veröffentlichungstag und Bekanntmachung des Hinweises auf die Patenterteilung:
**10.08.2016 Patentblatt 2016/32**

(51) Int Cl.:
*C07C 2/78* *(2006.01)*  *C07C 11/24* *(2006.01)*
*B01J 4/00* *(2006.01)*  *B01J 19/24* *(2006.01)*
*C01B 3/36* *(2006.01)*

(21) Anmeldenummer: **11782589.3**

(22) Anmeldetag: **09.11.2011**

(86) Internationale Anmeldenummer:
**PCT/EP2011/069697**

(87) Internationale Veröffentlichungsnummer:
**WO 2012/062784 (18.05.2012 Gazette 2012/20)**

(54) **VERFAHREN UND VORRICHTUNG ZUR HERSTELLUNG VON ACETYLEN UND SYNTHESEGAS**

METHOD AND DEVICE FOR PRODUCING ACETYLENE AND SYNGAS

PROCÉDÉ ET DISPOSITIF POUR LA PRÉPARATION D'ACÉTYLÈNE ET DE GAZ DE SYNTHÈSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.11.2010 US 412415 P**

(43) Veröffentlichungstag der Anmeldung:
**18.09.2013 Patentblatt 2013/38**

(73) Patentinhaber: **BASF SE**
**67056 Ludwigshafen am Rhein (DE)**

(72) Erfinder:
- **GROßSCHMIDT, Dirk**
  **68259 Mannheim (DE)**
- **RUSS, Michael**
  **67354 Römerberg (DE)**
- **RENZE, Peter**
  **68163 Mannheim (DE)**
- **VICARI, Maximilian**
  **67117 Limburgerhof (DE)**
- **WEICHERT, Christian**
  **67098 Bad Dürkheim (DE)**
- **EHRHARDT, Kai Rainer**
  **67346 Speyer (DE)**
- **NEUHAUSER, Horst**
  **67373 Dudenhofen (DE)**
- **ZAPF, Hans**
  **67112 Mutterstadt (DE)**
- **HAYES, Michael L. Gonzales**
  **Louisiana 70737 (US)**

(74) Vertreter: **BASF IP Association**
**BASF SE**
**ZRX-C6**
**67056 Ludwigshafen (DE)**

(56) Entgegenhaltungen:
**DE-A1- 4 422 815  US-A- 2 672 488**
**US-A- 3 240 836**

## Beschreibung

Die vorliegende Erfindung betrifft ein verbessertes Verfahren zur Herstellung von Acetylen und Synthesegas durch partiellen Oxidation von Kohlenwasserstoffen in einem Reaktor, bei welchem man dem Reaktor einen den Kohlenwasserstoff enthaltenden Strom sowie einen den Sauerstoff enthaltenden Strom zuleitet sowie eine Vorrichtung zur Durchführung des erfindungsgemäßen Verfahrens.

Hochtemperaturreaktionen zur partiellen Oxidation von Kohlenwasserstoffen werden üblicherweise in einem Reaktorsystem aus Mischeinheit, Brenner und Quencheinrichtung durchgeführt. Als Beispiel für eine solche Partialoxidation im Hochtemperaturbereich ist die Herstellung von Acetylen und Synthesegas durch partielle Oxidation von Kohlenwasserstoffen zu nennen. Diese wird beispielsweise in DE 875198, DE 1051845, DE1057094 und DE 4422815 beschrieben. Die US 2,672,488 A beschreibt eine Vorrichtung und ein Verfahren zur partiellen Oxididation von Kohlenwasserstoffen.

Hierin werden die für das BASF-Sachsse-Bartholome-Acetylenverfahren üblicherweise eingesetzten Mischer/Brennerblock/Feuerraum/Quench-Kombinationen - im Folgenden, wenn auf die Kombination Bezug genommen wird, vereinfacht als "Reaktor" bezeichnet - erläutert.

Die Ausgangsstoffe wie beispielsweise Erdgas und Sauerstoff werden dabei getrennt augeheizt, üblicherweise bis hin zu 600°C. In einer Mischzone werden die Reaktanden intensiv vermischt und nach Durchströmen eines Brennerblocks zur exothermen Reaktion gebracht. Der Brennerblock besteht in diesen Fällen aus einer bestimmten Anzahl aus parallelen Kanälen, in denen die Strömungsgeschwindigkeit der zündfähigen Sauerstoff/Erdgas-Mischung höher ist als die Flammengeschwindigkeit (Reaktionsgeschwindigkeit, Umsetzungsgeschwindigkeit), um ein Durchschlagen der Flamme in den Mischraum zu verhindern. Der metallische Brennerblock wird gekühlt, um den thermischen Belastungen standzuhalten. Je nach Aufenthaltszeit im Mischraum entsteht die Gefahr der Vor- und Rückzündung aufgrund der begrenzten thermischen Stabilität der Mischungen. Hier wird der Begriff der Zündverzugszeit, bzw. Induktionszeit gebraucht als die Zeitspanne, in der eine zündfähige Mischung keine nennenswerte intrinsische thermische Veränderung durchläuft. Die Induktionszeit ist abhängig von der Art der eingesetzten Kohlenwasserstoffe, dem Mischungszustand, von Druck und Temperatur. Sie bestimmt die maximale Aufenthaltszeit der Reaktanden im Mischraum. Reaktanden wie Wasserstoff, Flüssiggas oder Leichtbenzin, deren Einsatz aufgrund von Ausbeute- und /oder Kapazitätssteigerungen im Syntheseprozess besonders wünschenswert ist, zeichnen sich durch eine vergleichsweise hohe Reaktivität und damit geringe Induktionszeit aus.

Die im derzeitigen Produktionsmaßstab eingesetzten Acetylenbrenner zeichnen sich durch ihre zylinderförmige Geometrie des Feuerraums aus. Der Brennerblock weist vorzugsweise hexagonal angeordnete Durchführungsbohrungen auf. In einer Ausführungsform sind z.B. 127 Bohrungen á 27 mm Innendurchmesser hexagonal auf einem kreisförmigen Grundquerschnitt mit Durchmesser von ca. 500 mm angeordnet. In der Regel liegen die eingesetzten Kanaldurchmesser bei etwa 19 bis 27 mm Durchmesser. Der anschließende Feuerraum, in der die Flamme der acetylenbildenden partiellen Oxidationsreaktion stabilisiert wird, ist ebenfalls von zylindrischem Querschnitt, ist wassergekühlt und entspricht im Erscheinungsbild dem eines kurzen Rohres (von z.B. 180 bis 533 mm Durchmesser und 380 bis 450 mm Länge). In Höhe der feuerraumseitigen Oberfläche des Brennerblocks wird sogenannter Hilfssauerstoff dem Reaktionsraum zugeführt. Dadurch wird für eine Flammenstabilisierung und somit für einen definierten Abstand der Flammenwurzel und damit des Reaktionsbeginns zum Reaktionsabbruch durch die Quencheinrichtung gesorgt. Der gesamte Brenner aus Brennerblock und Feuerraum wird in einen Quenchbehälter größeren Querschnitts über einen Flansch von oben eingehängt. Auf Höhe der Austrittsebene aus dem Feuerraum sind außerhalb von dessen Umfang Quenchdüsen auf einem oder mehreren Quenchverteilerringen installiert, die das Quenchmedium, z.B. Wasser oder Öl, mit oder ohne Zuhilfenahme eines Zerstäubungsmedium zerstäuben und näherungsweise senkrecht zur Hauptströmungsrichtung der den Feuerraum verlassenden Reaktionsgase eindüsen. Dieser direkte Quench hat die Aufgabe, die reagierende Strömung extrem schnell auf ca. 100 °C (Wasserquench) und 200 °C (Ölquench) abzukühlen, so dass Folgereaktionen, d.h. insbesondere der Abbau von gebildetem Acetylen, eingefroren werden. Die Reichweite und Verteilung der Quenchstrahlen ist dabei idealerweise so bemessen, dass eine möglichst homogene Temperaturverteilung in möglichst kurzer Zeit erreicht wird.

Die im aktuellen Produktionsmaßstab eingesetzten Acetylenbrenner zeichnen sich durch eine zylinderförmige Geometrie des Feuerraums aus. Die Einsatzstoffe werden über einen Diffusor vorgemischt und unter Vermeidung von Rückvermischung dem Brennerblock über hexagonal angeordnete Durchführungsbohrungen zugeführt. Bei den bekannten Verfahren erfolgt die Vormischung der Einsatzstoffe im Mischdiffusor in einem relativ großen Volumen und unter hohen Vorwärmtemperaturen.

Bei dem beschriebenen technischen Verfahren entsteht neben Acetylen im Wesentlichen Wasserstoff, Kohlenmonoxid und Ruß. Die in der Flammenfront gebildeten Rußpartikel können als Keime an den Feuerraum-Seitenwänden (Feurrauminnenwänden) anhaften, worauf es zum Aufwachsen, Ablagern und Anbacken von Koksschichten kommt, wodurch das Verfahren in seiner Effektivität nachhaltig beeinträchtigt wird.

In den bestehenden Produktionsverfahren mit Öl- und Wasserquench werden diese Ablagerungen periodisch im Bereich der Feuerrauminnenwände mittels einer Stocherreinrichtung mechanisch abgereinigt. Hierfür ist eine aufwändige Steuerung der Stochereinrichtung nötig (Ullmann's Encyclopedia of Industrial Chemistry, 5th Edition, Volume A1, pages 97 - 144) und zudem wird die jeweilige Einsatzzeit des Mechanismus durch die thermische Belastung im Brennraum limitiert.

Es hat nicht an Versuchen gefehlt, den Nachteil des Anbackens von Koksschichten an der Feuerrauminnenwand zu vermeiden. So offenbart die Lehre der DE 2307300 das Injizieren einer gasförmigen Substanz in den Reaktor in einer Region zwischen maximaler Temperatur und Ort des Abschreckens (Anspruch 1). Hierbei soll es zu Reaktionen zwischen den zugegebenen Gasen und freien Radikalen kommen, wodurch die Koksbildung reduziert werden soll (Beschreibung, Seite 8, zweiter Absatz).

In der DE 3904330 A1 wird ein Verfahren zur Herstellung von Acetylenruß durch thermische Zersetzung von Acetylen beschrieben. Es wird hier bei diesem Verfahren, welches sich von dem Verfahren zur Herstellung von Acetylen deutlich unterscheidet (z.B. keine partielle Oxidation), erwähnt, dass man ggf. einen Inertgasstrom einleitet.

Die DE 1148229 beschreibt ein Verfahren zum Betreiben von Spaltkammern zur Behandlung von Kohlenwasserstoffen, wobei eine Spülung mit Wasserdampf vorgesehen ist und eine Kühlung der Wand zu einem Wasserschleier führen soll (Anspruch 1). Genauere Angaben über die Art der Ausführung der Spülung werden nicht gemacht. Es handelt sich bei dem vorgestellten Verfahren nicht um eine partielle Oxidation (POx), als Spülmedium wird flüssiges Wasser eingebracht und ein zusätzliches Beimischen eines Oxidationsmittels (z.B. Sauerstoff) zum Spülmedium ist nicht vorgesehen. Desweiteren wird im axialen Verlauf der Spaltkammer nur an maximal einer Stelle ein Spülmedium injiziert.

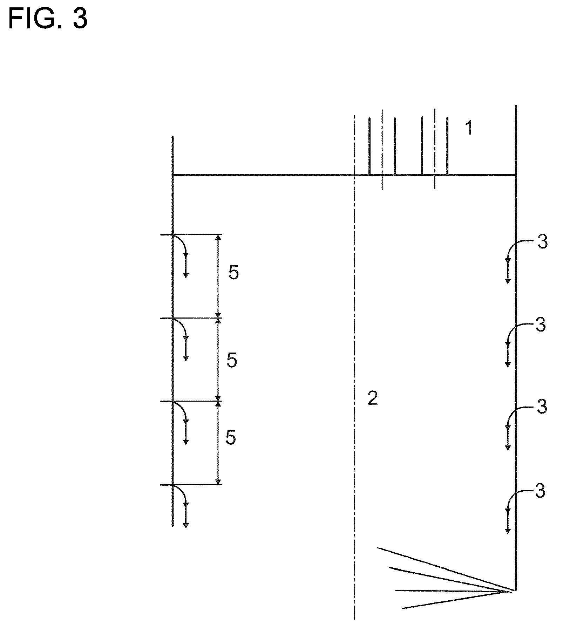

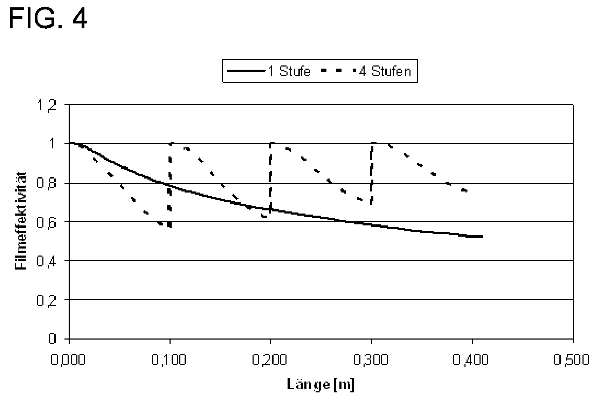

Gemäß der Lehre der DE 1250424 wird ein Verfahren zur Herstellung von Acetylen offenbart, bei welchem Wasserdampf an der Innenwand entlang geführt wird. Es fehlen jedoch genauere Angaben über konkrete Ausgestaltungsformen. Weiterhin wird zwar auch eine mehrstufige Zuführung eines Spülstromes in den Figuren 3 und 4 offenbart, diese Lösungen weisen jedoch über die Höhe der Brennkammer eine signifikante Veränderung des freien Querschnitts des Brennraumes auf, in Strömungsrichtung des Produktes betrachtet ist hier eine deutliche Vergrößerung zu betrachten. Dies führt jedoch in der Reaktion zu Inhomogenitäten durch verstärkte Rückvermischung im Feuerraum, wodurch die Effektivität des Verfahrens beeinträchtigt wird. Zudem wird das Spülmedium gemäß dieser Lehre auf 600 - 1000 °C überhitzt, was zu signifikanten Problemen bei der Materialbeständigkeit der Zuleitungen und zu erhöhten Bereitstellungskosten führt. Es handelt sich zudem um ein anderes Verbrennungskonzept der gestuften Verbrennung mit "Cracked-Gas"-Einspritzung (keine herkömmliche POx nach BASF-Sachse-Bartholome). Die Fluidgeschwindigkeit der Spülung übersteigt gemäß Vorschrift die mittlere Rauchgasgeschwindigkeit im Reaktor, was in der Grenzschicht zwischen beiden Strömungen zu Inhomogenitäten und damit sogar zur Ansaugung von Kokspartikeln auf die Feuerrauminnenwand führen kann. Die Feuerraumwand hat gemäß dieser Lehre im verfahrensgemäßen Betrieb eine Temperatur von etwa 700 °C was ebenfalls zu Problemen der Materialbeständigkeit-/Auswahl führen kann.

In der DE 2007997 wird beschrieben, wie ein Ölfilm an der Innenwand der Reaktionskammer eine Verkokung vermeiden soll (Seite 2, erster Absatz). Ein Ölfilm in einem Feuerraum neigt per se jedoch zur Verkokung. Daher kann ein kohlenwasserstoffhaltiges (Mineral-)Öl als Spülmedium bei der vorliegenden Herausforderung ausgeschlossen werden.

Die in den genannten Schriften offenbarten Verfahren zur Verhinderung bzw. Verringerung von unerwünschter Koksbildung befriedigen jedoch nicht bezüglich einer effektiven Anwendung beim Verfahren zur Herstellung von Acetylen. So beziehen sich einige der Schriften wie erläutert auf andere Reaktionen, wo gänzlich andere Bedingungen vorliegen und eine Übertragbarkeit nicht gegeben ist. So ist die bei dem erfindungsgemäßen Verfahren vorliegende partielle Oxidation in ihrer Charakteristik sehr anspruchsvoll: die Verweilzeiten spielen eine besonders große Rolle, der Abbruch der Reaktion muß sehr exakt erfolgen und die Zugabe von Fremdstoffen wie auch beispielsweise eines Spülgases oder Oxidators kann die Reaktion hinsichtlich ihres Ortes sowie ihrer Geschwindigkeit sehr schnell verschieben und damit zu einem Ausbeuteverlust führen.

Weiterhin finden sich im Stand der Technik nur allgemeine, eher aufgabenhafte Aussagen über die technische Ausgestaltung.

Weiterhin werden keine strömungsmechanischen und reaktionstechnischen Geometrievorschriften vorgestellt anhand derer eine effektive Minimierung des Spülmediums und damit eine Minimierung von Betriebskosten und eine Optimierung der Produktausbeute erfolgen könnten.

Es stellte sich somit die Aufgabe, ein verbessertes Verfahren zur partiellen Oxidation von Kohlenwasserstoffen zu finden, welches in verfahrenstechnisch einfacher Art und Weise Anbackungen und Ablagerungen an der Feuerrauminnenwand unterbindet, damit sich ein mechanisches Säubern dieser Feuerrauminnenwände erübrigt und somit eine periodische Abreinigung mittels thermisch hochbelasteter und aufwändig zu steuernden mechanischen Stochereinrichtungen entfällt.

Demgemäß wurde ein Verfahren zur Herstellung von Acetylen und Synthesegas durch partielle Oxidation von Kohlenwasserstoffen mit Sauerstoff gefunden, wobei die Ausgangsgase umfassend einen kohlenwasserstoffhaltigen Strom und einen sauerstoffhaltigen Strom zunächst getrennt vorerhitzt und anschließend in einer Mischzone vermischt werden und nach Durchströmen des Brennerblocks in dem Feuerraum zur Reaktion gebracht und anschließend schnell abgekühlt werden, welches dadurch gekennzeichnet ist, dass man die Feuerrauminnenwand mit einem Spülgasstrom belegt, man diesen Spülgasstrom mittels mehrerer Zuleitungen einbringt und man jede dieser Zuleitungen im Innern des Feuerraumes so ausbildet, dass die Orientierung des Richtungsvektors der Hauptströmung des aufgegebenen Spülgasstromes mit der Orientierung des Richtungsvektors der Hauptströmungsrichtung des durch den Brennerblock zugeführten Gasstromes in einem Winkel von maximal 10° abweicht und die Zuleitungen an ihrer Austrittsöffnung eine Spaltbreite von 1/1000 bis 3/100, bevorzugt 1/500 bis 1/100 des Feurraumdurchmessers aufweisen, und wobei in Bezug auf Hauptströmungsrichtung des durch den Brennerblock zugeführten Gasstromes betrachtet eine mehrstufige Zufuhr des Spülgasstromes an hintereinander liegenden Stellen erfolgt, wobei der freie Querschnitt des Feuerraumes, welcher dem aus dem Brennerblock austretenden Gasstrom zur Durchströmung des Feuerraumes zur Verfügung steht, auf Höhe der Zuleitungen des Spülgasstromes konstant ist, oder wobei eine Abweichung der aus dem Feuerraumdurchmesser resultierenden freien Querschnittsfläche weniger als 10 % bezogen auf die maximale freie Querschnittsfläche im Innern des Brennraumes beträgt.

Das erfindungsgemäße Verfahren lässt sich auf allgemein bekannte Verfahren zur Herstellung von Acetylen und/oder Synthesegas durch partielle Oxidation anwenden. Als Einsatzstoffe eigenen sich bei den Kohlenwasserstoffen bevorzugt u.a. Alkane, Alkene, Erdgase, Leichtbenzin und Gemischen dieser mit beispielsweise $CO_2$, Synthesegas. Der sauerstoffhaltige Strom kann beispielsweise über Sauerstoff aber auch Gemische enthaltend Sauerstoff sowie beispielsweise $CO_2$, $H_2O$, $N_2$, und/oder Edelgasen, zugeführt werden.

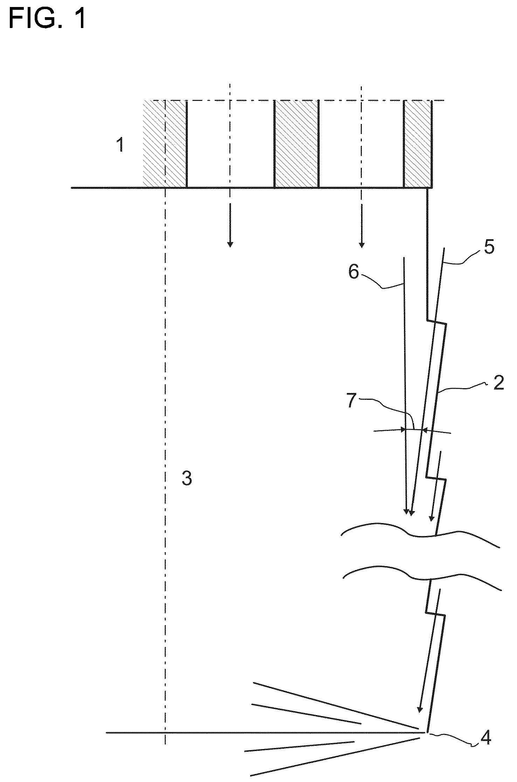

In Figur 1 sieht man einen Ausschnitt aus dem erfindungsgemäßen Brenner. Unter dem Feuerraum (3) im Sinne der Erfindung versteht man das rohrförmige Anlagenteil (welches in seinem Querschnitt z.B. kreis-, hexagon- oder rechteckförmig sein kann) stromab des Brennerblocks (1) und stromauf der Quencheinrichtung (4), welches stromauf einseitig durch die Brennerstirnfläche mit den Austrittlöchern des Brennerblocks begrenzt wird. Demgemäß beschreibt die Feuerrauminnenwand (2) die Innen- also die dem Reaktionsvolumen zugewendete - Fläche dieses Bauteils, auf welcher erfindungsgemäß eine Verkokung und Aufwachsung von Russ zu vermeiden ist.

Erfindungsgemäß wird der hierzu eingesetzte Spülgasstrom über mindestens zwei Zuleitungen zugeführt, wie sie beispielhaft in Figur 1 dargestellt sind. Hierbei ist es von Bedeutung, dass der Spülgasstrom in seiner Strömungsrichtung mit der des zugegebenen Kohlenwasserstoffs bzw. sauerstoffhaltigen Stromes im Wesentlichen übereinstimmt. Beispielhaft sei dies an Figur 1 erläutert: Der Einsatzstoffstrom durchströmt den Feuerraum (3) in mehr oder weniger vertikaler Richtung von oben nach unten. Auch wenn selbstredend an gewissen Rändern aufgrund von Realeffekten Abweichungen von dieser Richtung bestehen, ist somit die Hauptströmungsrichtung dieses Gasstromes symbolisiert durch den Richtungsvektor (6) (im Folgenden "Richtungsvektor 1" genannt) vertikal nach unten orientiert. Generell versteht man im Sinne dieser Erfindung unter "Richtung der Hauptströmung" jene Strömungsrichtung, welche sich in dem durchströmten Bauteil oberhalb der in Nähe der Wandung ausgeprägten Grenzschicht integral betrachtet ausbildet. Als für die Erfindung wesentliches Merkmal erfolgt die Zuleitung des Spülgasstromes bevorzugt so, dass die Richtungsvektoren der Hauptströmungsrichtungen von Spülgasstrom (5) und dem durch den Brennerblock dem Feuerraum zugeführten Gasstrom parallel sind. Auch der Richtungsvektor des Spülstromes (im Folgenden "Richtungsvektor 2" genannt) bezieht sich hierbei auf die Hauptströmungsrichtung des Spülmediums, wobei auf die vorstehenden Erläuterungen zum Begriff der "Hauptströmung" Bezug genommen wird. Die für die Erfindung wesentliche Parallelität dieser beiden Richtungsvektoren wird dadurch gewährleistet, dass die Flächen der Austrittsöffnungen der Zuleitungen in etwa senkrecht zum Richtungsvektor 1, bevorzugt senkrecht zu diesem Richtungsvektor angeordnet sind. Wie bereits erwähnt, kann es aufgrund von in der Realität zu beobachtenden Inhomogenitäten, nicht zu vermeidenden Fertigungstoleranzen und anderen Phänomenen dazu kommen, dass keine vollständige Parallelität der beiden Hauptströmungen der geschilderten Gasströme (symbolisiert durch die Richtungsvektoren 1 und 2) gegeben ist. Doch auch bei einer nur annährenden Parallelität können die erfindungsgemäßen positiven Effekte signifikant beobachtet werden, bevorzugt ist eine annähernde Parallelität erfindungsgemäß gegeben wenn der Winkel (7) zwischen den beiden Richtungsvektoren 1 und 2 ist kleiner als maximal 10°, besonders bevorzugt kleiner 5° ist, idealerweise ist eine vollständige Parallelität gewährleistet.

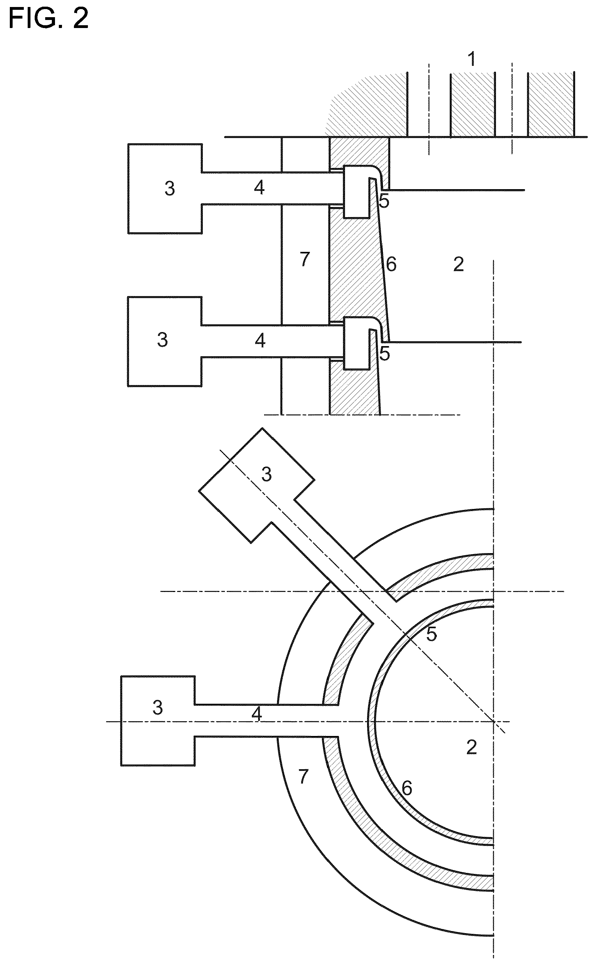

Es ist ein wesentliches Ziel des erfindungsgemäßen Verfahrens, die Menge des einzubringenden Spülstromes möglichst gering zu halten, um einen vorzeitigen Reaktionsabbruch zu verhindern, wodurch die Acetylenausbeute verringert werden würde. Zudem ist die Menge des eingesetzten Spülmediums aus Gründen der Wirtschaftlichkeit zu minimieren. Hierbei soll gleichzeitig eine hohe Effektivität bei der Verhinderung der Verkokung der Wandung des Feuerraumes erhalten bleiben. Um diesen Effekt zu erzielen ist ein möglichst gleichmäßiger Spülfilm auf der gesamten Feuerrauminnenwand zu gewährleisten. Ein Beispiel für eine konstruktive Lösung findet sich in Figur 2. Hier sieht man wiederum einen Ausschnitt aus einer erfindungsgemäßen Vorrichtung, wobei zwei unterschiedliche Schnitte dargestellt sind. Bei dieser effektiven konstruktiven Lösung erfolgt die Zuleitung des Spülgasstromes durch mehrere in axialer Richtung hintereinander angeordnete bevorzugt den gesamten Feuerraumumfang umfassende, ringspaltförmige Zuleitungen (5) in den Feuerraum (2). Um eine möglichst über den Feuerraumumfang gleichmäßig verteilte Menge an Spülmediums in jedem dieser Ringspalte zu gewährleisten, wird jeder Ringspalt durch mehrere über den Umfang gleichmäßig verteilte Zufuhrrohre (4) aus einem Verteilerreservoir (3) gespeist. Bevorzugt empfehlen sich eins bis acht Zuleitungen, welche gleichmäßig über den Umfang des Reaktors verteilt sind. Weiterhin ist in dieser Figur ein Teil des Brenners (1), die Feuerrauminnenwand (6) sowie ein Kühlmantel (7) zu sehen.

Bevorzugt leitet man dem dem zylindrisch geformten Feuerraum den Spülgasstrom über den Umfang gleichmäßig verteilt derart zu, dass der Spülgasstrom mit eins bis acht Zuleitungen eingebracht wird.

Hierbei können die Austrittsöffnungen der Zuleitungen einer Stufe ("Zuleitungsstufe") auf gleicher Höhe innerhalb der Feuerraumes angeordnet sein. Es kann sich dabei besonders empfehlen, die Zuleitung des Spülstromes über die Höhe des Feuerraumes mittels mehrerer solcher vorstehend geschilderter Zuleitungsstufen zu bewirken, wobei der Abstand zwischen den einzelnen Zuleitungsstufen 2 cm bis 30 cm, besonders bevorzugt 7 cm bis 20 cm beträgt.

Die Austrittsöffnungen der Zuleitungen des Spülstromes können bevorzugt kreisförmig, rechteckig oder quadratisch geformt sein bzw. einen den Umfang des Feuerraums umfassenden Ringspalt bilden.

Die Zuleitungen des Spülgasstromes (bevorzugt Ringspalte) weisen relativ kleine Öffnungen auf. Ihre Spaltbreite beträgt dabei etwa 1/1000 bis 3/100 , bevorzugt 1/500 bis 1/100 des Feuerraumdurchmessers. Unter dem Feuerraum im Sinne der Erfindung versteht man das rohrförmige Anlagenteil (welches in seinem Querschnitt z.B. kreis-, hexagon- oder rechteckförmig sein kann) stromab des Brennerblocks und stromauf der Quencheinrichtung, welches stromauf einseitig durch die Brennerstirnfläche mit den Austrittlöchern des Brennerblocks begrenzt wird. Demnach versteht man unter dem Feuerraumdurchmesser die größtmögliche radiale bzw. orthogonal zur Feuerrauminnenwand stehende Strecke, die die Feuerrauminnenwand(/wände) verbindet. Ist diese Strecke unabhängig von der axialen Erstreckung des rohrförmigen Feuerraums konstant, so ist im Sinne der Erfindung von einem konstanten Feuerraumdurchmesser zu sprechen. In der Regel liegen die Feuerraumdurchmesser etwa in einem Bereich von 10 bis 2000 mm, bevorzugt von 150 bis 1000 mm. Unter Spaltbreite versteht man in diesem Zusammenhang die maximale radiale Abmessung eines Auslasses des Spülmediums in den Feuerraum. Im Falle einer Zuleitung mit kreisförmigen Querschnitt handelt es sich somit um den Kreisdurchmesser und im Falle eines besonders bevorzugten Ringspalts um die Spaltbreite. In der Regel beträgt die Spaltbreite 0,2 bis 10mm, bevorzugt 0,3 bis 5 mm.

Erfindungsgemäß befinden sich die Austrittsöffnungen der Zuleitungen in unmittelbarer Nähe zu der zu spülenden Feurrauminnenwand (so beispielsweise in Figur 2 zu sehen). Bevorzugt gestaltet man die Positionierung der Zuleitungen des Spülgasstromes so, dass der Abstand des Flächenschwerpunkt der Austrittsöffnung der Zuleitungen von der Feuerrauminnenwand des Brennraumes weniger als 10 mm, bevorzugt weniger als 5 mm, besonders bevorzugt weniger als 1 mm beträgt. Die fluiddynamische Grenzschichtdicke über der Feuerrauminnenwand ergibt sich nach vereinfachten Annahme einer ebenen Platte nach der Lösung der Grenzschichtgleichung nach Blasius für die Lauflänge über der Feuerrauminnenwand. Bei einer Lauflänge von 1 bis 10 cm ergibt sich nach diesem Modell eine Grenschichtdicke von 1,5 bis 5 mm. Es ergibt sich für die gegebenen Geometrieeigenschaften der Spülvorrichtung somit vorteilhafterweise eine Spülung der Grenzschicht der Hauptströmung im Feuerraum. Durch fehlendes Eindringen und Quervermischen mit der Hauptströmung ergibt sich eine minimierte Menge des erforderlichen Spülstromes für eine effektive Reduzierung der Verkokung an der Wand, wodurch die Effektivität des Gesamtverfahrens deutlich erhöht wird, da mit der minimalen Menge an Spülmedium, zudem nur in Wandnähe eingebracht, der störende Einfluss auf die Zeitmaße der POx -Reaktionen ebenso minimiert wird.

Bei dem erfindungsgemäßen Verfahren kann das gewünschte Spülprinzip je nach eingesetztem Spülmedium auf zwei unterschiedlichen Mechanismen beruhen, welche auch beide gemeinsam zur Wirkung kommen können, wodurch sich besondere Synergismen ergeben.

Zum einen ein reines, wie zuvor beschriebenes, impulshaftes Spülen der Strömungsgrenzschicht. Dies führt zur Vermeidung sowohl des Eindringens von festen Koks-/Rußpartikeln als auch des Anhaftens und Aufwachsens von Ruß/Koksschichten. Die Geschwindigkeit des Spülfilms über der Spülfläche (Feurrauminnenwand) soll bevorzugt das 0,5 - 1,5 fache, besonders bevorzugt das 0,8 - 1 fache der Geschwindigkeit der Hauptströmung betragen.

Zum Zweiten besteht die Möglichkeit durch das Spülmedium in Wandnähe eine oxidierende Atmosphäre zu schaffen, welche ein Aufwachsen/Anhaften von Ruß/Koks dadurch vermeidet, dass mögliche Ablagerungen mit Hilfe des im Spülmedium enthaltenen Oxidators umgesetzt/verbrannt/oxidiert werden, bzw. deren Bildung soweit verlangsamt, dass eine Bildung der festen Phase aus kinetischen Gründen verhindert wird.

Bei dem erfindungsgemäßen Verfahren erfolgt die Zuleitung des Spülgasstromes über die Höhe des Reaktors an mindestens zwei hintereinander liegenden Stellen (im Folgenden jeweils als "Stufen" bezeichnet). Dies minimiert ebenfalls die einzusetzende Menge an Spülmedium, da durch stetige Erneuerung des Spülfilms in Wandnähe die Spüleffektivität erhöht wird. Unter Höhe des Feuerraums ist hierbei von der Orientierung die Ausdehnung in Hauptströmungsrichtung der durch den Brennerblock zugeführten Gasströme zu verstehen. Hierdurch wird die Effektivität des Verfahrens weiter gesteigert, da somit einer Verdünnung des Spülfilms durch Spaltgaseinmischung vermeiden werden kann und die Spülwirkung kann so über der gesamten Feuerraumlänge bei niedriger Dosierung des Spülmediums gewährleistet werden. Erfindungsgemäß kann in einer bevorzugten Ausführungsform die Spülstrommenge der Verkokungsneigung im jeweiligen Feuerraumabschnitt somit stufenlos angepasst werden.

Ein Beispiel für eine solche Realisierung ist in Figur 3 exemplarisch dargestellt. Die Abstände der einzelnen Zuleitungsstufen können gleich oder unterschiedlich sein, bevorzugt liegen sie in einem Bereich von 2 cm bis 30 cm, besonders bevorzugt 7 cm bis 20 cm. Der Abstand zwischen den einzelnen Stufen wird hierbei über den Abstand der beiden Austrittsöffnungen für das Spülmedium ermittelt. Innerhalb einer Stufe befinden sich die Zuleitungen in etwa auf einer Höhe, besonders bevorzugt auf gleicher Höhe. In Figur 3 sieht man den Ausschnitt eines Brenners (1) mit Feuerraum (2), wobei mehrere Zuleitungen für das Spülmedium (3) einen Spülfilm (4) auf der Feuerrauminnenwand aufbauen. Weiterhin ist der Abstand (5) zwischen zwei Spülstufen (Zuleitungsstufen) gekennzeichnet.

Es ist ein weiteres Merkmal des erfindungsgemäßen Verfahrens, dass trotz der mehrstufigen Zugabe des Spülgasstromes der Feuerraumdurchmesser über die Höhe annähernd konstant, bevorzugt konstant ist. Hierdurch werden in der Reaktion, welche wie ja bereits vorstehend geschildert sehr empfindlich auf Abweichungen reagiert, konstante Bedingungen gewährleistet und die Effektivität des Verfahrens weiter gesteigert. Eine Abweichung der aus dem Feuerraumdurchmesser resultierenden freien Querschnittsfläche von weniger als 10% bezogen auf die maximale freie Querschnittsfläche im Innern des Brennraumes kann hierbei toleriert werden, ohne dass die erfindungsgemäße Verbesserung so nachhaltig beeinträchtigt werden dass nicht der positive Effekt überwiegt und gilt im Rahmen dieser Erfindung als etwa konstant.

Erfindungsgemäß kann zur Erzeugung des Spülimpulses jedwelches Spülmedium in Betracht kommen, bevorzugt Wasserdampf. Die Temperaturen des Spülmediums liegen bevorzugt unter 500°C, besonders bevorzugt unter 200 °C. Hierbei beziehen sich die genannten Temperaturen auf die Eintrittstemperatur des Spülmediums in den Feuerraum. Bei der zuvor beschriebenen oxidativen Spülwirkung kommen bevorzugt als Spülgas bei gegebenen verfahrenstechnischen Randbedingungen potentiell oxidierende Medien bevorzugt Wasser, Wasserdampf, Sauerstoff, CO2, CO und Gemische daraus sowie aus weiteren Inertanteilen in Betracht. Vorzuziehen sind dabei Wasserdampf, Sauerstoff oder Gemische hieraus, so kann sich beispielsweise ein Spülmedium, welches neben vorwiegend Wasserdampf auch Sauerstoff in einem Bereich von bis zu 10 Vol% enthält, besonders empfehlen.

In einer bevorzugten Ausführungsform des erfindungsgemäßen Verfahrens belegt man zusätzlich die feuerraumseitige Oberfläche des Brennerblocks mit einem Spülgasstrom. Hierdurch kann das Verfahren in seiner Effektivität noch weiter gesteigert werden, da somit auch der Verkokung dieser Oberfläche wirkungsvoll begegnet werden kann. Als Spülmedium eignen sich dabei im Wesentlichen ebenfalls die vorstehend genannten Einsatzstoffe.

Weiterer Gegenstand dieser Erfindung ist eine für die Durchführung des erfindungsgemäßen Verfahrens geeignete Vorrichtung, wie sie in Figur 3, welche bereits zuvor erläutert wurde, dargestellt ist. Man kann hier einen üblichen zylindrischen Reaktor (1) enthaltend einen zylindrischen Feuerraum (2) zur Acetylenherstellung erkennen, wobei in dem Feuerraum mindestens zwei Zuleitungen (3) für einen Spülgasstrom derart angebracht sind, dass die Austrittsöffnungen der Zuleitungen senkrecht zur Achse des zylinderförmigen Feuerraumes stehen und die Austrittsöffnungen der Zuleitungen im Feuerraum in Nähe der Feuerrauminnenwand angeordnet sind, wobei der Flächenschwerpunkt der Austrittsöffnungen der Zuleitungen einen Abstand von der Feuerrauminnenwand von weniger als 10 mm, bevorzugt weniger als 1 mm aufweist.

Das erfindungsgemäße Verfahren bietet die Möglichkeit, Verkrustungen an der Feuerrauminnenwand zu vermeiden, wodurch mechanische Abreinigungstätigkeiten wie beispielsweise der Einsatz einer Stochereinrichtung vermieden werden können. Somit ist es dauerhaft gewährleistet, dass keine störenden Feststoffpartikel von relevanter Größe in den Gasstrom gelangen. Hierdurch ist es in einer bevorzugten Ausgestaltung des erfindungsgemäßen Verfahrens somit möglich, einen großen Teil der in dem Gas enthaltenen Wärme nach der Reaktion mittels eines nachgeschalteten Wärmetauschers zurückzugewinnen. Hierbei wird diese gewonnene Energie bevorzugt zur Dampferzeugung verwendet. Bevorzugt kann so aus dem hergestellten Produktgas ein Großteil der Wärme zur Dampferzeugung rückgewonnen werden. Als Wärmetauscher eigenen sich beispielsweise Rohrbündelwärmetauscher. Bevorzugt wird hier der Gasstrom nach der Reaktion im Gegensatz zum herkömmlichen Verfahren nicht auf 100 °C bei Wasserquench bzw. 200 °C bei Ölquench, sondern schnell auf 600 - 1000 °C abgekühlt (gequencht). Dies führt noch zu keinen nennenswerten Ausbeuteverlusten an Acetylen durch Folgereaktionen. Anschließend wird dieses heiße Produktgas einem Wärmetauscher zugeführt, in welchem das Gas weiter abgekühlt wird. Die dabei freiwerdende Energie wird zur Wärmerückgewinnung verwendet. Bevorzugt handelt es sich bei dem Wärmetauscher um einen Dampferzeuger, in dem wird indirekt das Produktgas weiter abgekühlt und sekundärseitig Dampf erzeugt, welcher weiter kommerziell oder prozesstechnisch nutzbar ist. Hierdurch wird die Effektivität des Verfahrens deutlich gesteigert. Es ist ein wesentlicher Vorteil bei der Ausgestaltung des erfindungsgemäßen Verfahrens, dass der Wärmetauscher unmittelbar nachgeschaltet werden kann, da hierbei keine Feststoffablagerungen zu Problemen führen. Neben einer Verwendung der bei der Abkühlung des Produktgases freiwerdenden Energie zur Dampferzeugung sind auch andere Möglichkeiten gegeben, diese Energie im Rahmen des Verfahrens zur Herstellung von Acetylen und Synthesegas zu nutzen.

Eine solche Verfahrensführung ist bei den bisherigen Verfahren so nicht wirtschaftlich realisierbar, da sich beim bisherigen mechanischen Abreinigungsverfahren Ruß- und Koksplatten im Zuge der Abreinigung von der Feuerrauminnenwand lösen, in Richtung Wärmetauscher transportiert werden und diesen mechanisch, abrasiv schädigen oder ganz oder teilweise verstopfen. Demgegenüber lässt sich erfindungsgemäß eine Ablagerung von Koks und Ruß auf der Feuerrauminnenwand vollständig unterbinden.

Das erfindungsgemäße Verfahren bietet eine verfahrenstechnisch einfache und effektive Möglichkeit zur wirkungsvollen Verringerung oder gar Verhinderung von Verkrustungen und Anbackungen von Koks an der Feuerrauminnenwand bei der Herstellung von Acetylen und / oder Synthesegas durch partielle Oxidation. Durch die vorstehend erläuterten, erfindungsgemäßen Merkmale kann hierbei bereits ein relativ geringer Spülgasstrom die Verkrustung wirkungsvoll verhindern, ohne dass die Effektivität der Reaktion nachhaltig beeinträchtigt wird. Die mehrstufige Ausgestaltung der Zufuhr des Spülmediums reduziert den Bedarf an Spülmedium zusätzlich und hierdurch kann der Bedarf an Spülmedium vorteilhafterweise in den einzelnen Zonen gezielt zugegeben werden. Durch entsprechende Auswahl des Spülmediums kann neben einem "reinen Spülvorgang" basierend auf Impuls des Spülstroms ggf. auch bevorzugt eine oxidierende Atmosphäre geschaffen werden, wodurch die Ablagerungen weiter verringert werden können.

Beispiele

In den folgenden drei Beispielen soll hier der Vergleich zwischen dem Betrieb eines Brennerblock / Feuerraums / Quenchs zur Acetylensynthese ("Standardreaktor") nach Stand der Technik und dem Betrieb eines Reaktors im Sinne der Erfindung betrachtet werden.

Beispiel 1

Der Standardreaktor wurde zu Testzwecken mit herkömmlicher axialer und radialer Flammenstabilisierung betrieben. Der Feuerraum ist wassergekühlt ausgeführt.

Der Reaktordurchmesser beläuft sich bei den verwendeten Brennern auf 180 mm.
Der Brennerblock ist ausgeführt mit 19 hexagonal angeordneten Bohrungen a 27mm Innendurchmesser.

Der Standardreaktor und alle im Folgenden genannten Reaktoren wurden unter diesen Reaktionsbedingungen betrieben:

- Erdgasvolumenstrom: 600 $Nm^3/h$
- Verhältnis Sauerstoffstrom zu Erdgasstrom = 0.62

- Vorwärmtemperatur der Gase: 550°C
- Acetylen im Volumen: 6.4%vol

Unter den gegebenen Versuchsbedingungen musste der Standardreaktor nach dem Auftreten von Flammenstabilitätsproblemen nach ca. 20 Betriebsstunden abgeschaltet werden.
Bei Inspektion des Feuerraums ist beim Standardreaktor eine ausgebildete, mehrere Zentimeter dicke Koksschicht auf der Feuerrauminnenwand auffindbar.
Die Aufwachsrate der Koksschicht kann zu 4mm/h bestimmt werden.

Beispiel 2

Zum Vergleich wurde ein erfindungsgemäßer Reaktor mit Zuleitungen eines Spülstroms in Form eines Ringspaltes mit einer Breite von 1/200 des Feuerraumdurchmessers eingesetzt. Dieser beträgt auch in diesem Beispiel 180mm. Die Zuleitungen der Spülpositionen wurden mit dem Spülgas zum impulshaften Spülen der Spülgrenzschicht durchströmt.
Die Spülmenge wurde so bemessen, dass die Geschwindigkeit des Spülfilms das 0.8fache der Hauptströmung beträgt.
Beim Betrieb des erfindungsgemäßen Reaktors kommt es im Feuerraum während einer Betriebszeit von 20h zu keinen Ablagerungen an der Feuerrauminnenwand.
Die Aufwachsrate der Koksschicht beträgt demnach 0.

Beispiel 3

Ein wie in Beispiel 2 betriebener erfindungsgemäßer Reaktor wurde mit Zugabe eines Oxidators über die Spülpositionen so betrieben, dass sich in der Spülgrenzschicht in Nähe der Feuerrauminnenwand eine oxidative Atmosphäre ausbildet. Auch bei diesem Betrieb werden Ablagerungen dauerhaft verhindert.
Die Aufwachsrate der Koksschicht beträgt auch hier 0.

Beispiel 4:

Ein wesentliches Merkmal des erfindungsgemäßen Verfahrens ist die Spülung der Feuerrauminnenwand und damit die Vermeidung von Anbackungen und Verkokungen. Dies geschieht wie bereits erläutert erfindungsgemäß am effektivsten indem ein möglichst dünner Spülfilm in bereits erläutertem Geschwindigkeits- und Richtungsverhältnis zur Hauptströmung in den Feuerraum über Zuleitungen mit im Zuge der Beschreibung bereits dargelegten Geometrieeigenschaften eingebracht wird. Ein wichtiges erfindungsgemäßes Detail ist es hierbei den gesamten Spülstrom nicht an einer Stelle des Feuerraums einzubringen, sonder den Spülstrom aufzuteilen und über mehrere hintereinander angeordnete Stufen in erfindungsgemäßen Abständen dem Feuerraum zuzuführen. Dies führt zum einen bei gleicher eingebrachter Menge an Spülmedium zu einer Erhöhung der Filmeffektivität in der mehrstufigen Variante im Vergleich zur einstufigen Variante. Zum anderen lässt sich die gleiche integrale Filmeffektivität bei mehrstufiger Ausführung mit weniger Spülmedium erzielen, als bei einstufiger Ausführung.

Die Filmeffektivität im Sinne der Erfindung ist dabei wie folgt zu definieren. Sie Repräsentiert den Quotient der Konzentrationsdifferenz des zu Spülenden Mediums auf der Wand (Index W) und dem Hauptstrom (Index m) geteilt durch die Konzentrationsdifferenz im Spülgasstrom (Index C) und der Hauptströmung (Index m):

$$\eta = \frac{C_w - C_m}{C_c - C_m}$$

Ein Berechnungsmodell für die Filmeffektivität bezüglich des zu spülenden Mediums in Abhängigkeit der axialen Lauflänge x nach der Eindüsung des Spülmediums lautet nach [1]:

$$\eta = A \left\{ \frac{x}{Ms} \left[ \text{Re}_c \, \frac{\mu_c}{\mu_m} \right]^{-0.25} \right\}^{-0.8}$$

Die Konstante A repräsentiert dabei gemäß [1] die experimentspezifischen geometrischen und verfahrenstechnischen Randbedingungen.

Dabei gilt:

M - Massenstromverhältnis
s - Spaltbreite
Rec - Reynoldszahl
- dyn. Viskosität
A - Konstante aus Experiment
Index c - Spülfilm
Index m - Hauptströmung

In der nachfolgenden Tabelle ist vergleichend die hieraus ermittelte Filmeffektivität für einen konstanten Gesamtspülstrom aufgeteilt auf 4 Stufen im erfindungsgemäßen Fall und aufgeteilt auf nur eine Stufe im zweiten Fall (gemäß Stand der Technik) dargestellt. Daraus lassen sich die erfindungsgemäßen Vorteile der mehrstufigen Spülung erkennen. Demnach ergibt sich für die erfindungsgemäße vierstufige Variante eine Steigerung der integralen über die Feuerraumoberfläche gemittelten Filmeffektivität von 20 % gegenüber der einstufigen Variante bei gleichem Gesamtspülstrom der in den Feuerraum eingebracht wurde. In Figur 4 ist ein graphischer Vergleich der beiden Filmeffektivitäten dargestellt. Es ist hier die Filmeffektivität über de Länge (in m) aufgetragen. Die durchgezogene Linie stellt den Fall für eine Stufe dar, die gestrichelte Linie den erfindungsgemäßen Fall für vier Stufen.

Als Simulationssoftware wurde ANSYS FLUENT Flow Modeling Software Version 6.3 Benutzt.

[2] Burns, W. K. and Stollery, J.L., The influence of foreign gas injection and slot geometry on film cooling effectiveness, Int. J. Heat Mass Transfer, Vol. 12, S. 935-951

Tabelle: Beispiel - Vergleich ein- und vierstufige Spülung der Feuerrauminnenwand Massenstrom: 0,052 kg/s

eingedüst über eine und vier Stufen

|  | Filmeffektivität | Filmeffektivität |
| --- | --- | --- |
| Länge [m] | 1 Stufe | 4 Stufen |
| 0,00 | 1 | 1 |
| 0,05 | 0,88683844 | 0.739 |
| 0,10 | 0,77715877 | 0,544 |
| 0,10 | 0,777158774 | 1 |
| 0,15 | 0,715233613 | 0,798 |
| 0,20 | 0,662728085 | 0,64 |
| 0,20 | 0,662728085 | 1 |
| 0,25 | 0,584392286 | 0,84 |
| 0,30 | 0,584392286 | 0,702 |
| 0,30 | 0,584392286 | 1 |
| 0,35 | 0,549715748 | 0,861 |
| 0,40 | 0,523118006 | 0,744 |

Für eine Stufe beträgt der integrale Mittelwert η= 0,69, für vier Stufen beträgt der integrale Mittelwert η= 0,83, dies entspricht einer Steigerung von ca. 20%.

**Patentansprüche**

1. Verfahren zur Herstellung von Acetylen und Synthesegas durch partielle Oxidation von Kohlenwasserstoffen mit Sauerstoff, wobei die Ausgangsgase umfassend einen kohlenwasserstoffhaltigen Strom und einen sauerstoffhaltigen Strom zunächst getrennt vorerhitzt und anschließend in einer Mischzone vermischt werden und nach Durchströmen des Brennerblocks in dem Feuerraum zur Reaktion gebracht und anschließend schnell abgekühlt werden, **dadurch gekennzeichnet, dass** man die Feuerrauminnenwand mit einem Spülgasstrom belegt, man diesen Spülgasstrom mittels mehrerer Zuleitungen einbringt und man jede dieser Zuleitungen im Innern des Feuerraumes so ausbildet, dass die Orientierung des Richtungsvektors der Hauptströmung des aufgegebenen Spülgasstromes mit der Orientierung des Richtungsvektors der Hauptströmungsrichtung des durch den Brennerblock zugeführten Gasstromes in einem Winkel von maximal 10° abweicht und die Zuleitungen an ihrer Austrittsöffnung eine Spaltbreite von 1/1000 bis 3/100, bevorzugt 1/500 bis 1/100 des Feuerraumdurchmessers aufweisen, wobei in Bezug auf die Hauptströmungsrichtung des durch den Brennerblock zugeführten Gasstromes betrachtet eine mehrstufige Zufuhr des Spülgasstromes an hintereinander liegenden Stellen erfolgt, wobei der freie Querschnitt des Feuerraumes, welcher dem aus dem Brennerblock austretenden Gasstrom zur Durchströmung des Feuerraumes zur Verfügung steht, auf Höhe der Zuleitungen des Spülgasstromes konstant ist, oder wobei eine Abweichung der aus dem Feuerraumdurchmesser resultierenden freien Querschnittsfläche weniger als 10 % bezogen auf die maximale freie Querschnittsfläche im Innern des Brennraumes beträgt.

2. Verfahren nach Anspruch 1, **dadurch gekennzeichnet, dass** die Richtungsvektoren der Hauptströmungsrichtungen von Spülgasstrom und des durch den Brennerblock dem Feuerraum zugeführten Gasstromes parallel sind.

3. Verfahren nach Ansprüchen 1 oder 2, **dadurch gekennzeichnet, dass** man dem zylindrisch geformten Feuerraum den Spülgasstrom über den Umfang gleichmäßig verteilt derart zuleitet, dass der Spülgasstrom mit eins bis acht Zuleitungen eingebracht wird.

4. Verfahren nach Anspruch 3, **dadurch gekennzeichnet, dass** die Austrittsöffnungen der Zuleitungen einer Stufe auf gleicher Höhe innerhalb der Feuerraumes angeordnet sind.

5. Verfahren nach Anspruch 4, **dadurch gekennzeichnet, dass** die Zuleitung des Spülstromes über die Höhe des Feuerraumes mittels mehrerer Zuleitungsstufen gemäß Anspruch 4 erfolgt, wobei der Abstand zwischen den einzelnen Zuleitungsstufen 2 cm bis 30 cm, besonders bevorzugt 7 cm bis 20 cm beträgt.

6. Verfahren nach Ansprüchen 1 bis 5, **dadurch gekennzeichnet, dass** die Austrittsöffnungen der Zuleitungen des Spülstromes kreisförmig, rechteckig oder quadratisch sind bzw. einen den Umfang des Feuerraums umfassenden Ringspalt bilden.

7. Verfahren nach Ansprüchen 1 bis 6, **dadurch gekennzeichnet, dass** man als Spülmedium Wasserdampf, Sauerstoff oder Gemische davon einsetzt

8. Verfahren nach Ansprüchen 1 bis 7, **dadurch gekennzeichnet, dass** das Spülmedium bis zu 10 Vol% Sauerstoff enthält.

9. Verfahren nach Ansprüchen 1 bis 8, **dadurch gekennzeichnet, dass** der Flächenschwerpunkt der Austrittsöffnungen der Zuleitungen einen Abstand von der Feurrauminnenwand von weniger als 10 mm, bevorzugt weniger als 1 mm aufweist.

10. Verfahren nach Ansprüchen 1 bis 9, **dadurch gekennzeichnet, dass** man das Gas nach der Reaktion in dem Feuerraum auf eine Temperatur von 600 bis 1000°C abkühlt und anschließend einem Wärmetauscher zuführt, in welchem das Gas weiter abgekühlt wird und die freiwerdende Energie zur Wärmerückgewinnung verwendet wird.

11. Vorrichtung zur Durchführung eines der Verfahren gemäß Ansprüchen 1 bis 10, welche einen zylindrischen Reaktor enthaltend einen zylindrischen Feuerraum zur Acetylenherstellung umfasst, wobei in dem Feuerraum mindestens zwei Zuleitungen für einen Spülgasstrom derart angebracht sind, dass die Flächen der Austrittsöffnungen der Zuleitungen senkrecht zur Achse des zylinderförmigen Feuerraumes stehen und die Austrittsöffnungen der Zuleitungen im Feuerraum in Nähe der Feuerrauminnenwand angeordnet sind, wobei der Flächenschwerpunkt der Austrittsöffnungen der Zuleitungen einen Abstand von der Feuerrauminnenwand von weniger als 10 mm, bevorzugt weniger als 1 mm aufweist.

**Claims**

1. A process for preparing acetylene and synthesis gas by partial oxidation of hydrocarbons with oxygen, by first separately preheating the starting gases comprising a hydrocarbon-containing stream and an oxygen-containing stream and then mixing them in a mixing zone and, after they have flowed through the burner block, reacting them in the firing space and then cooling the products rapidly, wherein the firing space interior wall is blanketed with a purge gas stream, this purge gas stream is introduced by means of a plurality of feed lines and each of these feed lines is configured in the interior of the firing space such that the alignment of the direction vector of the main flow of the purge gas stream introduced deviates with the alignment of the direction vector of the main flow direction of the gas stream supplied through the burner block at an angle of not more than 10°, and the feed lines at the exit orifice thereof have an opening width of 1/1000 to 3/100, preferably 1/500 to 1/100, of the firing space diameter, there being a multi-level supply of the purge gas stream at successive points viewed in relation to the main flow direction of the gas stream supplied through the burner block, and the free cross section of the firing space which is available to the gas stream leaving the burner block for flow through the firing space at the height of the feed lines of the purge gas stream being constant, or with a deviation of the free cross-sectional area resulting from the firing space diameter of less than 10% based on the maximum free cross-sectional area in the interior of the combustion space.

2. The process according to claim 1, wherein the direction vectors of the main flow directions of purge gas stream and of the gas stream supplied through the burner block to the firing space are parallel.

3. The process according to claim 1 or 2, wherein the purge gas stream is supplied to the cylindrically shaped firing space in homogeneous distribution over the periphery in such a way that the purge gas stream is introduced with one to eight feed lines.

4. The process according to claim 3, wherein the exit orifices of the feed lines of one level are arranged at the same height within the firing space.

5. The process according to claim 4, wherein the purge stream is fed in over the height of the firing space by means of a plurality of feed line levels according to claim 4, the distance between the individual feed line levels being 2 cm to 30 cm, more preferably 7 cm to 20 cm.

6. The process according to claims 1 to 5, wherein the exit orifices of the feed lines of the purge stream are circular, rectangular or square, or form an annular gap surrounding the circumference of the firing space.

7. The process according to claims 1 to 6, wherein the purge medium used is steam, oxygen or mixtures thereof.

8. The process according to claims 1 to 7, wherein the purge medium comprises up to 10% by volume of oxygen.

9. The process according to claims 1 to 8, wherein the area centroid of the exit orifices of the feed lines has a distance from the firing space interior wall of less than 10 mm, preferably less than 1 mm.

10. The process according to claims 1 to 9, wherein the gas, after the reaction in the firing space, is cooled to a temperature of 600 to 1000°C and then supplied to a heat exchanger in which the gas is cooled further, and the energy released is used for heat recovery.

11. An apparatus for performing any of the processes according to claims 1 to 10, which comprises a cylindrical reactor comprising a cylindrical firing space for acetylene preparation, at least two feed lines for a purge gas stream being mounted within the firing space such that the faces of the exit orifices of the feed lines are at right angles to the axis of the cylindrical firing space, and the exit orifices of the feed lines in the firing space are arranged close to the firing space interior wall, and the area centroid of the exit orifices of the feed lines having a distance from the firing space interior wall of less than 10 mm, preferably less than 1 mm.

**Revendications**

1. Procédé de fabrication d'acétylène et d'un gaz de synthèse par oxydation partielle d'hydrocarbures avec de l'oxygène, les gaz de départ qui comprennent un courant contenant des hydrocarbures et un courant contenant de l'oxygène étant tout d'abord préchauffés séparément, puis mélangés dans une zone de mélange, et mis en réaction dans la chambre de combustion après avoir traversé le bloc brûleur, puis rapidement refroidis, **caractérisé en ce que** la paroi intérieure de la chambre de combustion est recouverte avec un courant de gaz de rinçage, ce courant de gaz de rinçage étant introduit par plusieurs conduites d'alimentation et chacune de ces conduites d'alimentation étant configurées à l'intérieur de la chambre de combustion de sorte que l'orientation du vecteur directionnel de l'écoulement principal du courant de gaz de rinçage introduit diffère à un angle d'au plus 10° avec l'orientation du vecteur directionnel de la direction de l'écoulement principal du courant gazeux introduit par le bloc brûleur, et les conduites d'alimentation présentant au niveau de leur ouverture de sortie une largeur de fente de 1/1 000 à 3/100, de préférence de 1/500 à 1/100 du diamètre de la chambre de combustion, une introduction en plusieurs étapes du courant de gaz de rinçage ayant lieu à des emplacements successifs par rapport à la direction de l'écoulement principal du courant gazeux introduit par le bloc brûleur, la section transversale libre de la chambre de combustion, qui est à la disposition du courant gazeux sortant du bloc brûleur pour traverser la chambre de combustion, étant constante à la hauteur des conduites d'alimentation du courant de gaz de rinçage, ou une déviation de la surface de section transversale libre résultant du diamètre de la chambre de combustion étant de moins de 10 % par rapport à la surface de section transversale libre maximale à l'intérieur de la chambre de combustion.

2. Procédé selon la revendication 1, **caractérisé en ce que** les vecteurs directionnels des directions de l'écoulement principal du courant de gaz de rinçage et du courant gazeux introduit dans la chambre de combustion par le bloc brûleur sont parallèles.

3. Procédé selon la revendication 1 ou 2, **caractérisé**

**en ce que** le courant de gaz de rinçage est introduit dans la chambre de combustion de forme cylindrique réparti uniformément sur la périphérie, le courant de gaz de rinçage étant introduit avec une à huit conduites d'alimentation.

4. Procédé selon la revendication 3, **caractérisé en ce que** les ouvertures de sortie des conduites d'alimentation d'une étape sont agencées à la même hauteur dans la chambre de combustion.

5. Procédé selon la revendication 4, **caractérisé en ce que** l'introduction du courant de rinçage a lieu sur la hauteur de la chambre de combustion au moyen de plusieurs étapes d'alimentation selon la revendication 4, l'écart entre les étapes d'alimentation individuelles étant de 2 cm à 30 cm, de manière particulièrement préférée de 7 cm à 20 cm.

6. Procédé selon les revendications 1 à 5, **caractérisé en ce que** les ouvertures de sortie des conduites d'alimentation du courant de rinçage sont circulaires, rectangulaires ou carrées, ou forment une fente annulaire comprenant la périphérie de la chambre de combustion.

7. Procédé selon les revendications 1 à 6, **caractérisé en ce que** de la vapeur d'eau, de l'oxygène ou leurs mélanges sont utilisés en tant que milieu de rinçage.

8. Procédé selon les revendications 1 à 7, **caractérisé en ce que** le milieu de rinçage contient jusqu'à 10 % en volume d'oxygène.

9. Procédé selon les revendications 1 à 8, **caractérisé en ce que** le barycentre géométrique des ouvertures de sortie des conduites d'alimentation présente un écart avec la paroi intérieure de la chambre de combustion de moins de 10 mm, de préférence de moins de 1 mm.

10. Procédé selon les revendications 1 à 9, **caractérisé en ce que** le gaz est refroidi à une température de 600 à 1 000 °C après la réaction dans la chambre de combustion, puis introduit dans un échangeur de chaleur dans lequel le gaz est davantage refroidi, et l'énergie libérée est utilisée pour la récupération de chaleur.

11. Dispositif pour la réalisation d'un des procédés selon les revendications 1 à 10, qui comprend un réacteur cylindrique contenant une chambre de combustion cylindrique pour la fabrication d'acétylène, au moins deux conduites d'alimentation pour un courant de gaz de rinçage étant disposées dans la chambre de combustion de sorte que les surfaces des ouvertures de sorties des conduites d'alimentation soient perpendiculaires à l'axe de la chambre de combustion cylindrique, et les ouvertures de sortie des conduites d'alimentation soient agencées dans la chambre de combustion à proximité de la paroi intérieure de la chambre de combustion, le barycentre géométrique des ouvertures de sortie des conduites d'alimentation présentant un écart avec la paroi intérieure de la chambre de combustion de moins de 10 mm, de préférence de moins de 1 mm.

FIG. 1

FIG. 2

FIG. 3

# FIG. 4

**IN DER BESCHREIBUNG AUFGEFÜHRTE DOKUMENTE**

**In der Beschreibung aufgeführte Patentdokumente**

- DE 875198
- DE 1051845
- DE 1057094
- DE 4422815
- US 2672488 A
- DE 2307300
- DE 3904330 A1
- DE 1148229
- DE 1250424
- DE 2007997

**In der Beschreibung aufgeführte Nicht-Patentliteratur**

- Ullmann's Encyclopedia of Industrial Chemistry. vol. A1, 97-144
- **BURNS, W. K. ; STOLLERY, J.L.** The influence of foreign gas injection and slot geometry on film cooling effectiveness. *Int. J. Heat Mass Transfer,* vol. 12, 935-951